# FASCICULE DE BREVET EUROPEEN

(11) **EP 2 142 916 B1**
(45) Date de publication et mention de la délivrance du brevet: **08.08.2018**
(21) Numéro de dépôt: 08736390.9
(22) Date de dépôt: 18.04.2008
(51) Int. Cl.: G01N 27/22, G01N 33/03, A47J 37/12

(54) **DISPOSITIF DE MESURE CAPACITIVE DE LA QUALITÉ ET/OU DE LA DÉGRADATION D'UN FLUIDE, COMPORTANT UN CAPTEUR CAPACITIF DÉCOUPLÉ MÉCANIQUEMENT DE SON ENCAPSULATION**
KAPAZITIVE MESSVORRICHTUNG ZUR PRÜFUNG DER QUALITÄT UND/ODER VERSCHLECHTERUNG EINES FLUIDES, MIT EINEM VON SEINEM GEHÄUSE ENTNEHMBAREN KAPAZITIVEN SENSOR
DEVICE FOR THE CAPACITIVE MEASUREMENT OF THE QUALITY AND/OR DEGRADATION OF A FLUID, INCLUDING A CAPACITIVE SENSOR THAT IS MECHANICALLY DECOUPLED FROM THE ENCAPSULATION ELEMENT THEREOF

(30) Priorité: 20.04.2007 CH 653072007
(43) Date de publication de la demande: 13.01.2010
(73) Titulaire: Alpsens Technologies Inc., 1024 Ecublens (CH)
(72) Inventeur: CHAMBON, Gérald, CH-1018 Lausanne (CH)
(74) Mandataire: Ravenel, Thierry Gérard Louis
(86) Numéro de dépôt international: PCT/EP2008/054745
(87) Numéro de publication internationale: WO 2008/135367

(56) Documents cités:
- EP-A- 1 588 158
- WO-A-2005/098406
- JP-A- 61 044 339
- US-A- 5 573 650
- US-A1- 2004 175 992
- US-B1- 6 469 521
- TESTO: "Cooking Oil Tester with Display and Alarm"[Online] février 2004 (2004-02), pages 1-2, XP002488531 Extrait de l'Internet: URL:http://www.appleonehk.com/265_E.pdf> [extrait le 2008-07-16]
- "Praxis-Fibel Frittierölmessung", , 1 January 2006 (2006-01-01), pages 1-63, XP55229501, Retrieved from the Internet: URL:http://documents.rs-components.com/EIT C/DE/messtech/fett_fibel.pdf [retrieved on 2015-11-19]

## Description

La présente invention concerne un dispositif de mesure capacitive de la qualité et/ou de la dégradation d'un fluide, notamment d'une huile. L'invention concerne en particulier un tel dispositif comportant un capteur capacitif permettant la mesure de la qualité et/ou de la dégradation d'une huile de friture, directement disposé dans l'appareil de cuisson, et dans lequel le capteur capacitif encapsulé dans un boîtier de protection fixé dans une zone immergée de la cuve de l'appareil de cuisson est découplé mécaniquement de son encapsulation.

Il est bien connu que les huiles alimentaires se dégradent lors de la cuisson, notamment lorsqu'elles sont portées de manière répétée à des températures élevées. Typiquement, pour frire des aliments ces huiles sont portées à des températures de l'ordre de 180°C. A ces températures se produisent une multitude de réactions chimiques, telles que des polymérisations, des thermo-oxydations, etc., qui altèrent de manière importante la qualité de l'huile. La quantité de certains produits des ces réactions ne doit pas dépasser un seuil imposé par la législation, car au-delà de ce seuil l'huile est considérée comme impropre à la consommation. Il est donc important de pouvoir détecter ce seuil de manière fiable afin de remplacer l'huile dès que cela est nécessaire. Pendant longtemps ce moment a été laissé à l'appréciation des cuisiniers qui suite à une inspection visuelle et/ou olfactive déterminaient si l'huile était encore propre à la consommation. Bien entendu un telle méthode est purement subjective et n'est par conséquent pas fiable.

Un dispositif de mesure capacitive de la qualité et/ou de la dégradation d'une huile de friture a été proposé le brevet EP 1 588 158 pour remédier à ces inconvénients. Dans ce dispositif, le capteur capacitif est directement disposé dans la cuve de l'appareil de cuisson, ce capteur étant encapsulé dans un boîtier de protection perforé fixé dans une zone immergée de la cuve.

Bien que le dispositif décrit dans cette demande de brevet fonctionne de manière satisfaisante il n'en demeure pas moins que la mesure de capacité à l'intérieur d'une friteuse est une opération très sensible. En effet, la variation de quelques picofarads entre de l'huile neuve et de l'huile usagée, fortement influencée par la température, l'eau et les impuretés n'est pas aisée à détecter. A cela s'ajoute le fonctionnement du dispositif dans un environnement très dur dans lequel le capteur capacitif, et le cas échéant le capteur de température auquel il est associé, sont soumis à des températures élevées supérieures à 200°C, à des chocs lorsque des opérateurs peu soigneux heurtent le capteur avec les paniers recevant les aliments à frire.

La protection du capteur capacitif, et le cas échéant celle du capteur de température auquel il est associé, constitue donc un problème extrêmement important qu'il convient d'adresser pour ne pas détériorer la précision des mesures et/péjorer la durée de vie du dispositif de mesure, car ces mesures en dépendent directement.

On connaît également d'un document TESTO intitulé « cooking oil tester with display and alarm » un dispositif de mesure capacitive de la qualité d'une huile de cuisson portable comprenant, dans une partie supérieure, un boitier logeant un dispositif d'affichage associé à une unité électronique de commande et de traitement, et dans sa partie inférieure un capteur capacitif attaché au boitier au moyen d'un tube traversé par des conducteurs électriques reliant le capteur à l'unité électronique de commande et de traitement. Ce capteur peut être équipé d'un simple capuchon de protection amovible rigidement fixé sur le capteur, ou d'un arceau de protection amovible qui est en place pendant la mesure. L'invention a donc pour but de remédier à ce problème en fournissant un dispositif de mesure capacitive de la qualité et/ou de la dégradation d'un fluide défini par la revendication 1 du brevet.

L'invention porte donc principalement sur le découplage entre le capteur capacitif - relativement fragile, assemblé et aligné avec précision - et son encapsulation. La fonction de cette dernière étant de protéger au mieux le capteur dans les situations suivantes :
Dans l'huile, en utilisation normale : protéger contre les chocs des paniers tout en permettant une circulation optimale de l'huile ainsi que des chocs dus à tout autre instrument

Hors cuve pour nettoyage : protection contre les chutes, les chocs dans la machine à laver, le transport, ...

Le découplage mécanique du capteur et de son encapsulation permet d'augmenter le taux de fiabilité (moins de pièces sous contrainte), de rallonger la durée de vie du capteur et de simplifier l'assemblage. Un autre avantage réside dans la séparation des fonctions de mesure et des fonctions d'encapsulation, ce qui permet donc :
- de sous-traiter une partie du système (encapsulation) tout en gardant la maîtrise des processus d'assemblage du capteur.
- d'apporter des modifications à l'une ou l'autre des parties sans affecter l'autre.
- de simplifier une grande partie des pièces de l'encapsulation en réduisant de manière importante les contraintes sur celle-ci et donc évidemment également les coûts :
- matériaux : stabilité thermiques et contraintes mécanique en température moins importantes
- tolérances d'usinage moins grandes car l'alignement n'a pas besoin d'être optimal.

D'autres caractéristiques et avantages de la présente invention apparaîtront dans la description suivante d'un mode de réalisation préféré d'un dispositif de mesure selon l'invention, présenté à titre d'exemple non limitatif en référence aux dessins annexés, dans lesquels :
- La figure 1 est une vue schématique en coupe d'une cuve de friteuse dans laquelle un dispositif de mesure avec son capteur encapsulé selon l'invention peut être monté;
- La figure 2 est une vue schématique du capteur encapsulé du dispositif selon l'invention

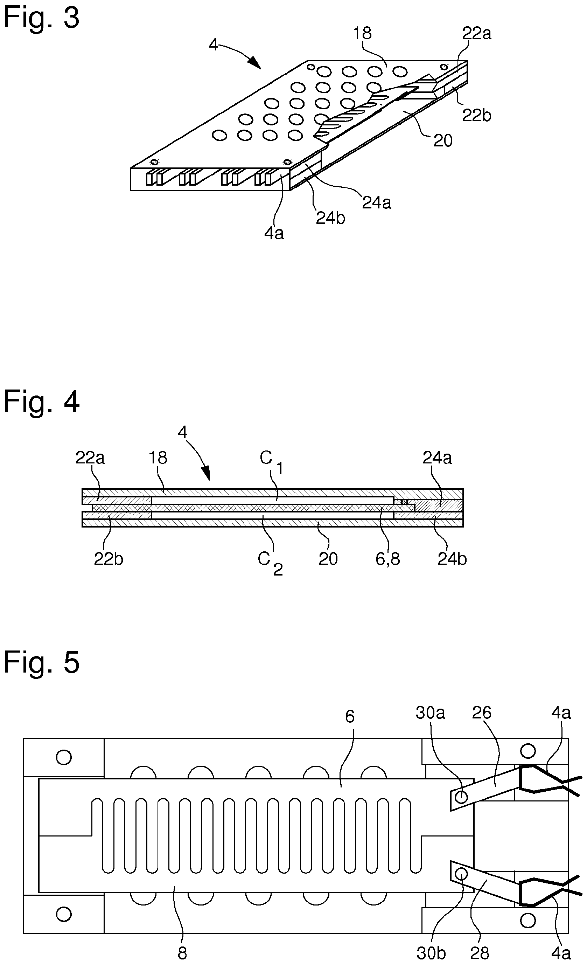

- La figure 3 est une vue schématique en perspective partiellement arrachée du capteur encapsulé du dispositif selon l'invention
- La figure 4 est une vue schématique en coupe du capteur encapsulé selon l'invention;
- La figure 5 est une vue schématique en élévation du capteur selon l'invention sur laquelle une plaque d'encapsulation a été omise;

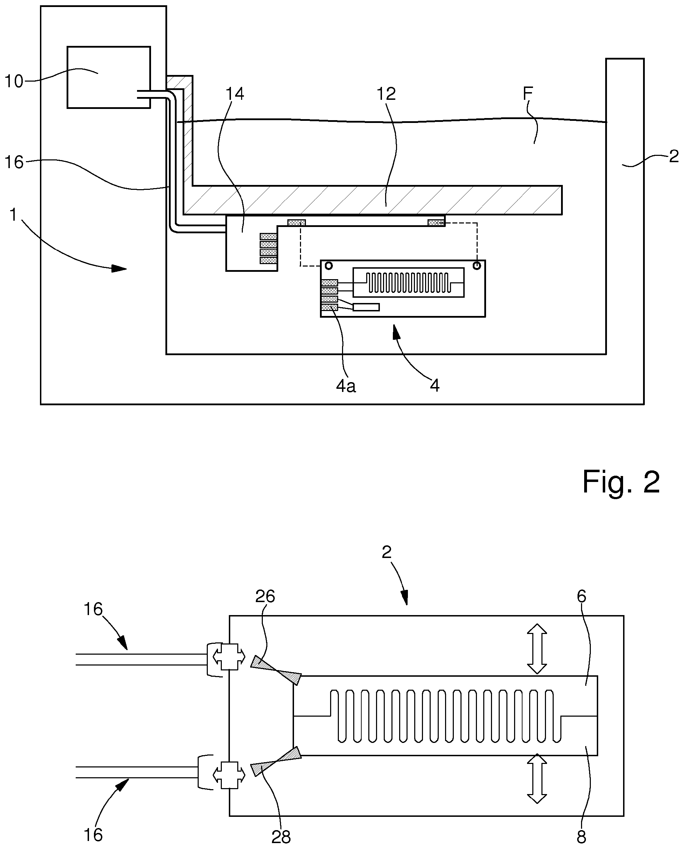

En se référant tout d'abord à la figure 1, on voit un mode de réalisation d'un dispositif de mesure capacitive de la qualité et/ou de la dégradation d'un fluide notamment d'une huile, désigné par la référence générale 1.

On notera que la description qui suit sera faite dans une application du dispositif 1 à la mesure de la qualité et/ou de la dégradation d'une huile alimentaire ou analogue, utilisé pour frire des aliments dans un appareil de cuisson comprenant une cuve 2 dans laquelle l'huile peut être chauffée typiquement jusqu'à environ 200°C.

Le dispositif de mesure 1 comprend un capteur encapsulé 4 comportant une paire d'électrodes 6, 8 espacées l'une de l'autre et destinées à être immergées dans un fluide F (Figure 2), par exemple l'huile d'une friteuse, dont on souhaite mesurer la qualité et/ou la dégradation et déterminer si elle est toujours propre à l'emploi. Les électrodes 6, 8 forment avec l'huile F un élément capacitif de mesure EFM dont la capacité varie en fonction de la constante diélectrique de l'huile. Lorsque l'huile se dégrade, la quantité de composés polaires présents dans celle-ci augmente et conduit à une augmentation de sa constante diélectrique. Ainsi, en mesurant l'évolution de la capacité de l'élément capacitif de mesure EFM, on peut déterminer le degré de qualité et/ou de dégradation de l'huile. Le capteur 4, et plus précisément son élément capacitif EFM, est donc capable de fournir un signal électrique de sortie représentatif de la constante diélectrique de l'huile sur une large plage de températures, en particulier entre 20°C et 200°C. Le signal électrique est traité par un circuit de traitement électronique 10 disposé à l'extérieur de la cuve 2. Le capteur 4 est relié au circuit de traitement électronique par des contacts électriques 4a. Le capteur est par exemple fixé de façon amovible sous le corps de chauffe 12 par l'intermédiaire d'un support de fixation et de connexion 14 solidaire de ce dernier. Typiquement le capteur 4 peut s'enficher dans le support 14 par ses contacts électriques 4a qui sont du par exemple conformée en pince élastiques. Le support de fixation et de connexion 14 est relié au circuit électronique au moyen de câbles 16 protégés, par exemple dans des tubes.

Chaque électrode 6, 8 de la paire présente la forme d'un peigne ayant une pluralité de dents sensiblement parallèles entre elles et s'étendant à partir d'une base. Les électrodes 6 et 8 sont disposées l'une par rapport à l'autre de sorte que les dents 6a d'une électrode 6 sont imbriquées entre les dents 8a de l'autre électrode 8. Les dents des électrodes 6 et 8 sont ainsi disposées sensiblement dans un même plan.

On notera à ce propos que les électrodes 6 et 8 sont par exemple formées à partir d'une même plaque plane découpée de façon appropriée, la plaque étant suffisamment rigide pour que les électrodes gardent leur forme lorsqu'elles sont manipulées. Dans l'exemple décrit, les électrodes sont réalisées à partir d'une plaque en un acier alimentaire (acier inox austénitique de type 18-10 à bas carbone) ayant une épaisseur comprise entre 0,1 et 3 mm. D'autres types d'aciers alimentaires peuvent être également utilisés par exemple le Z7CN18-09, le Z3CND18-12-02, Z6CNDT17-12 et Z7CNU16-04. La plaque est découpée au moyen d'un faisceau laser, ce qui permet de réaliser entre les dents des électrodes des entrefers compris entre 10 nm et 1 mm. Il est bien entendu que plus l'entrefer est faible, plus la sensibilité de l'élément capacitif est grande. Selon une variante de réalisation, on peut également envisager de réalisé des électrodes formées d'un substrat enrobé d'un matériau conducteur, par exemple un substrat enrobé d'une couche d'or de platine ou analogue.

Les électrodes 6 et 8 sont disposées dans un boîtier d'encapsulation perforé. Ce boîtier est formée de plaques 18, 20 planes perforées métalliques entre lesquelles s'étendent les électrodes 6, 8 avec interposition aux extrémités de deux paires d'entretoises 22a, 22b et 24a, 24b en matière isolante entre lesquelles les électrodes 6, 8 formant la sonde impédimétrique sont prises en sandwich. Les électrodes 6, 8 sont fixées aux plaques 18, 20 via les entretoises 22a, 22b à une extrémité et sont guidées libres dans les entretoises 24a, 24b à leur extrémité opposée.

Les perforations des plaques 18, 20 de l'encapsulation sont disposées en regard de la région de mesure des électrodes 6 et 8, c'est-à-dire en regard des entrefers définis par les espaces entre les dents 6a de l'électrode 6 et les dents 8a de l'électrode 8. Grâce à cette configuration, le fluide à mesurer, en l'occurrence l'huile, baigne les deux faces des électrodes 6 et 8 de part et d'autre du plan de ces électrodes de sorte qu'elle peut venir circuler au voisinage des dents 6a et 8a des électrodes 6 et 8.

Cette structure d'encapsulation des électrodes permet d'optimiser la circulation de l'huile autour des deux faces des électrodes planes et notamment de créer deux canaux C1, C2 définis respectivement entre une première surface des électrodes 6, 8 et la plaque perforée 18 et une deuxième surface des électrodes 6, 8 opposée à la première et la plaque perforée 20.

La fixation des électrodes 6 et 8 aux entretoises est réalisée par des moyens élastiques, à savoir par deux lames ressort 26, 28 assurant également la fonction de contact électrique entre les électrodes et des éléments de contact 4a du capteur 2.

Par cette fixation élastique on réalise un découplage mécanique du capteur de son boîtier d'encapsulation. Ces lames, découpées dans une feuille d'acier inox de 100 microns d'épaisseur par électro-érosion, assurent le positionnement élastique de la sonde dans le boîtier d'encapsulation. La sonde est guidée dans la direction perpendiculaire au plan des plaques 18 et 20 par éléments de fixation 30a, 30b qui se logent dans des alésages des entretoises 24a, 24b. Un petit jeu est laissé afin que la sonde soit « libre» dans la direction perpendiculaire au plan des plaques 18 et 20 dans son emplacement, reposant simplement sur les pièces isolantes.

Les entretoises sont réalisées de préférence en un matériau résistant à des températures comprises entre 20°C et 200°C et présentant un faible coefficient de dilatation thermique, tel qu'un matériau céramique. Cependant elles peuvent être réalisées en tout autre matériau isolant compatible avec l'application du dispositif de mesure envisagée. A titre d'exemple pour une application alimentaire devant être stable dans la gamme de température susmentionnée, les entretoises pourraient également être réalisées en un polymère fluoré tel que le Téflon.

## Revendications

1. Dispositif de mesure capacitive de la qualité et/ou de la dégradation d'un fluide comportant un capteur encapsulé dans un boîtier perforé formé de plaques planes perforées métalliques (18, 20 séparées par deux paires d'entretoises (22a, 22b, 24a ,24b) ledit capteur comprenant une paire d'électrodes (6,8) s'étendant entre lesdites plaques et qui sont reliées aux entretoises (22a, 22b, 24a ,24b) à une extrémité de manière à être découplées mécaniquement de celles-ci par des moyens de fixation élastiques formés par des lames ressorts (26, 28).

2. Dispositif de mesure selon la revendication 1, **caractérisé en ce que** lesdites lames ressort (26, 28) assurent également la fonction de contact électrique entre les électrodes du capteur et des éléments de contact du capteur destinés à connecter le capteur avec l'extérieur.

3. Dispositif de mesure selon la revendication 2 **caractérisé en ce que** les éléments de contact sont conformés en pinces élastiques.

4. Dispositif de mesure selon la revendication 2 ou 3 **caractérisé en ce que** les lames ressort assurant le découplage mécanique entre le capteur et son encapsulation sont réalisées en acier inox et présentent une épaisseur de l'ordre de 100 microns.

## Patentansprüche

1. Kapazitive Vorrichtung zum Messen der Qualität und/oder der Verschlechterung eines Fluids, umfassend einen Sensor, der in ein gelochtes Gehäuse eingekapselt ist, das aus ebenen metallischen Lochplatten (18, 20) gebildet ist, die durch zwei Paare Abstandhalter (22a, 22b, 24a, 24b) getrennt sind, wobei der Sensor ein Paar Elektroden (6, 8) aufweist, die sich zwischen den Platten erstrecken und die mit den Abstandhaltern (22a, 22b, 24a, 24b) an einem Ende in einer Weise verbunden sind, dass sie von diesen durch elastische Befestigungsmittel, die durch Federplättchen (26, 28) gebildet sind, mechanisch entkoppelt sind.

2. Messvorrichtung nach Anspruch 1, **dadurch gekennzeichnet, dass** die Federplättchen (26, 28) ferner die Funktion des elektrischen Kontakts zwischen den Elektroden des Sensors und Kontaktelementen des Sensors, die dazu bestimmt sind, den Sensor mit der äußeren Umgebung zu verbinden, sicherstellen.

3. Messvorrichtung nach Anspruch 2, **dadurch gekennzeichnet, dass** die Kontaktelemente als Federklemmen ausgebildet sind.

4. Messvorrichtung nach Anspruch 2 oder 3, **dadurch gekennzeichnet, dass** die Federplättchen, die die mechanische Entkopplung zwischen dem Sensor und seiner Einkapselung sicherstellen, aus Edelstahl hergestellt sind und eine Dicke in der Größenordnung von 100 µm aufweisen.

## Claims

1. Device for the capacitive measurement of the quality and/or deterioration of a fluid that includes a sensor encapsulated in a perforated case, formed of perforated flat metal plates (18, 20) separated by two pairs of spacers (22a, 22b, 24a, 24b), said sensor including a pair of electrodes (6,8) extending between said plates and which are connected to said spacers (22a, 22b, 24a, 24b) at one end so as to be mechanically uncoupled therefrom by elastic fixing means formed by strip springs (26, 28).

2. Measuring device according to claim 1, **characterized in that** said strip springs also fulfil the function of electrical contact between the sensor electrodes and contact elements of the sensor that will connect the sensor to the exterior.

3. Measuring device according to claim 2, **characterized in that** the contact elements take the form of elastic clamps.

4. Measuring device according to claim 2 or 3, **characterized in that** the strip springs ensuring the mechanical separation between the sensor and its encapsulating case are made of stainless steel and have a thickness of the order of 100 microns.
